# EUROPEAN PATENT APPLICATION

(11) **EP 1 681 064 A1**
(43) Date of publication of application: **19.07.2006**
(21) Application number: 05028331.6
(22) Date of filing: 24.07.2000
(51) Int. Cl.: A61K 39/39, A61P 37/04, A61K 38/21

(54) **Induction of antigen-specific T cells by interferon**

(30) Priority: 22.07.1999 JP 20768799
(62) Divisional of application: 00306263.5
(71) Applicant: Dainippon Sumitomo Pharma Co., Ltd., Osaka 541-8524 (JP)
(72) Inventor: Takasu, Hideo, Nishinomiya-shi Hyogo-ken (JP); Gotoh, Masashi, Takatsuki-shi Osaka 569-0081 (JP); Yamaoka, Takashi, Sanda-shi Hyogo-ken (JP)
(74) Representative: Duckworth, Timothy John

(57) **Abstract**

Use of
(1) at least one selected from the group consisting of antigenic proteins, antigenic peptides derived from the antigenic proteins, and DNAs capable of expressing said antigenic proteins or said antigenic peptides; and
(2) at least one selected from the group consisting of interferons and DNAs capable of expressing said interferons,
   in the manufacture of an agent for induction of antigen-specific T cells.

SEQUENCE LISTING

<110> Sumitomo Pharmaceuticals Co., Ltd.

<120> Agent for Induction of Antigen-Specific T Cell

<150> JP 11-207687
<151> 1999-07-22

<160> 34

<210> 1
<211> 9
<212> PRT
<213> Homo sapiens

<400> 1
Lys Gly Ser Gly Lys Met Lys Thr Glu
                5

<210> 2
<211> 9
<212> PRT
<213> Homo sapiens

<400> 2
Lys Leu Asp Glu Glu Ala Leu Leu Lys
                5

<210> 3
<211> 9
<212> PRT
<213> Homo sapiens

<400> 3
Val Leu Ser Gly Ser Gly Lys Ser Met
                    5

<210> 4
<211> 9
<212> PRT
<213> Homo sapiens

<400> 4
Glu Tyr Arg Gly Phe Thr Gln Asp Phe
                    5

<210> 5
<211> 9
<212> PRT
<213> Homo sapiens

<400> 5
Ala Phe Arg Gln Leu Ser His Arg Phe
                    5

<210> 6
<211> 11
<212> PRT
<213> Homo sapiens

<400> 6
Lys Met Lys Thr Glu Arg Arg Met Lys Lys Leu
                    5                   10

<210> 7
<211> 10
<212> PRT
<213> Homo sapiens

<400> 7
Arg Met Lys Lys Leu Asp Glu Glu Ala Leu
                    5                   10

<210> 8
<211> 11
<212> PRT
<213> Homo sapiens

<400> 8
Arg Met Lys Lys Leu Asp Glu Glu Ala Leu Leu
                    5                   10

<210> 9
<211> 9
<212> PRT
<213> Homo sapiens

<400> 9
Lys Met Ser Ser Ser Asp Thr Pro Leu
                    5

<210> 10
<211> 9
<212> PRT
<213> Homo sapiens

<400> 10
Leu Leu Leu Cys Gln Asn Lys Gly Leu
                    5

<210> 11
<211> 10
<212> PRT
<213> Homo sapiens

<400> 11
Leu Leu Leu Cys Gln Asn Lys Gly Leu Leu
                5                   10

<210> 12
<211> 9
<212> PRT
<213> Homo sapiens

<400> 12
Leu Leu Glu Thr Thr Val Gln Lys Val
                5

<210> 13
<211> 9
<212> PRT
<213> Homo sapiens

<400> 13
Arg Val Lys Ala Pro Asn Lys Ser Leu
                5

<210> 14
<211> 9
<212> PRT
<213> Homo sapiens

<400> 14
Tyr Val Asp Glu Thr Gly Arg Lys Leu
                5

<210> 15
<211> 9
<212> PRT
<213> Homo sapiens

<400> 15
Ala Gln Lys Thr Pro Tyr Ile Val Leu
                    5

<210> 16
<211> 9
<212> PRT
<213> Homo sapiens

<400> 16
Lys Phe His Arg Val Ile Lys Asp Phe
1                   5

<210> 17
<211> 9
<212> PRT
<213> Homo sapiens

<400> 17
Asp Phe Met Ile Gln Gly Gly Asp Phe
1                   5

<210> 18
<211> 10
<212> PRT
<213> Homo sapiens

<400> 18
Val Tyr Asp Tyr Asn Cys His Val Asp Leu
                    5                   10

<210> 19
<211> 10
<212> PRT
<213> Homo sapiens

<400> 19
Leu Phe Glu Lys Ala Val Lys Asp Tyr Ile
                5                   10

<210> 20
<211> 9
<212> PRT
<213> Homo sapiens

<400> 20
Asn Tyr Asn Lys Ala Leu Gln Gln Leu
                5

<210> 21
<211> 9
<212> PRT
<213> Homo sapiens

<400> 21
Ala Tyr Ile Asp Phe Glu Met Lys Ile
                5

<210> 22
<211> 10
<212> PRT
<213> Homo sapiens

<400> 22
Asp Tyr Val Glu Ile Trp Gln Ala Tyr Leu
                5                   10

<210> 23
<211> 9
<212> PRT
<213> Homo sapiens

<400> 23
Asp Tyr Leu Arg Arg Arg Val Asp Phe
                5

<210> 24
<211> 9
<212> PRT
<213> Homo sapiens

<400> 24
Ala Phe Thr Arg Ala Leu Glu Tyr Leu
                5

<210> 25
<211> 10
<212> PRT
<213> Homo sapiens

<400> 25
Gly Phe Asp Cys Ala Asn Glu Ser Val Leu
                5                   10

<210> 26
<211> 9
<212> PRT
<213> Homo sapiens

<400> 26
Glu Tyr Cys Leu Lys Phe Thr Lys Leu
                5

<210> 27
<211> 8
<212> PRT
<213> Homo sapiens

<400> 27
Leu Tyr Gln Ala Val Ala Thr Ile
                    5

<210> 28
<211> 9
<212> PRT
<213> Saccharomyces cerevisiae

<400> 28
Glu Tyr Arg Gly Phe Thr Pro Met Phe
                    5

<210> 29
<211> 9
<212> PRT
<213> Artificial Sequence
<220>
<221> VARIANT
<223> Designed peptide based on the motif for antigenic peptide from
      SART-1, to which HLA antigen binds.

<400> 29
Glu Tyr Arg Gly Phe Thr Gln Asp Ile
                    5

<210> 30
<211> 9
<212> PRT
<213> Artificial Sequence
<220>
<221> VARIANT
<223> Designed peptide based on the motif for antigenic peptide from
      SART-1, to which HLA antigen binds.

<400> 30

Glu Tyr Arg Gly Phe Thr Gln Asp Leu
                    5

<210> 31
<211> 9
<212> PRT
<213> Artificial Sequence
<220>
<221> VARIANT
<223> Designed peptide based on the motif for antigenic peptide from
      SART-1, to which HLA antigen binds.

<400> 31
Glu Tyr Arg Gly Phe Thr Gln Asp Trp
                    5

<210> 32
<211> 9
<212> PRT
<213> Artificial Sequence
<220>
<221> VARIANT
<223> Designed peptide based on the motif for antigenic peptide from
      cyclophilin B, to which HLA antigen binds.

<400> 32
Lys Tyr His Arg Val Ile Lys Asp Phe
 1                  5

<210> 33
<211> 9
<212> PRT
<213> Artificial Sequence
<220>
<221> VARIANT
<223> Designed peptide based on the motif for antigenic peptide from

cyclophilin B, to which HLA antigen binds.

<400> 33
Asp Tyr Met Ile Gln Gly Gly Asp Phe
1               5

<210> 34
<211> 9
<212> PRT
<213> Influenza virus

<400> 34
Ala Ser Asn Glu Ser Met Glu Thr Met
                5

## Description

The present invention relates to an agent for induction of antigen-specific T cell. More specifically, the present invention relates to an agent for induction of T cell specific to antigenic peptide derived from tumor or virus.

In the elimination of cancer cells and virus-infected cells by the living body, cellular immunity, especially antigen-specific T cell [cytotoxic T cell (hereinafter also referred to as "CTL") and helper T cell] plays a central role. Antigen-specific T cell recognizes cell surface MHC molecule (in the case of human, also referred to as "HLA molecule") bound to fragment peptide from antigenic protein derived from cancer or virus, by means of T cell receptor, specifically injures cancer cells and virus-infected cells and produces various cytokines to activate immunity. Fragment peptide presented to MHC molecule is called antigenic peptide and is usually about 8 to about 20 amino acids in length. The vaccine therapy in which cancer antigenic peptide or viral antigenic peptide is administered to a living body to enhance specific T cell in the living body is thought to be useful in the treatment and prophylaxis of cancers and viral infectious diseases. In order to efficiently induce specific immunity using a vaccine, it is effective to simultaneously administer an immune-activating substance, i.e., an adjuvant, along with the principal component antigenic protein or antigenic peptide. Commonly known adjuvants include aluminum compounds and molecules derived from bacteria (*FASEB*:6, 3265, 1996; *Ann. Rev. Immunol.*:**4** 369, 1986). In recent years, there have been elucidated that GM-CSF and IL-12 are effective as such vaccine adjuvants. In other words, there has been reported that GM-CSF and IL-12 are administered along with antigenic peptide in the preparation form of a water-in-oil emulsion to enhance the induction of antigenic peptide-specific CTL (*J. Immunol*., 158:3947, 1997). Furthermore, there has also been reported that GM-CSF is administered simultaneously with antigenic peptide to induce delayed-type hypersensitivity (DTH) to the same extent as with complete Freund's adjuvant, thereby being effective in inducing specific immunity *(Blood,* 88:202, 1996).

Interferons, cytokines produced by various cells, such as lymphocytes and fibroblasts, in a living body, exhibit antiviral action, anticancer action, etc., and have principal subtypes such as α, β and γ.

Interferon-α (hereinafter abbreviated as "IFN-α") is cytokine produced mainly by leukocytes and has been found as an antiviral protein produced by virus-infected cells (C.R. Seance, *Soc. Biol*., 152:1627, 1958). IFN-α has been known to possess various biological activities, including suppressive action of tumor cell growth, enhancement for cytotoxic activity of T cells and NK cells, and enhancement for expression of MHC class I, as well as antiviral action (*Immunol. Today,* 17:369, 1996). Regarding the induction of antigen-specific immunity, there is a report that IFN-α itself is effective in inducing antigen-specific antibodies (*J. Biol. Regul. Homest. Agents,* 8:9, 1994); however, little is known about other actions.

An object of the present invention is to provide an agent for induction of antigenic peptide-specific T cell that is effective in the treatment of tumors and viral infectious diseases.

The above and other objects of the present invention will be apparent from the following description.

In sum, the present invention relates to:
[1] an agent for induction of antigen-specific T cell comprising as active ingredients:
   (1) at least one selected from the group consisting of antigenic proteins, antigenic peptides derived from the antigenic proteins, and DNAs capable of expressing the antigenic proteins or the antigenic peptides; and
   (2) at least one selected from the group consisting of interferons and DNAs capable of expressing the interferons;
[2] an enhancing agent for induction of antigen-specific T cell comprising at least one selected from the group consisting of interferons and DNAs capable of expressing the interferons as active ingredients;
[3] a method for inducing antigen-specific T cell in an individual in need thereof, comprising administering the agent of item [1] above to the individual in therapeutically effective amounts;
[4] a method for enhancing induction of antigen-specific T cell in an individual in need thereof, comprising administering the enhancing agent of item [2] above to the individual in therapeutically effective amounts;
[5] use of the agent of item [1] above for inducing antigen-specific T cell in an individual in need thereof;
[6] use of the enhancing agent of item [2] above for enhancing induction of antigen-specific T cell in an individual in need thereof;
[7] a method for inducing antigen-specific T cell in an individual in need thereof, comprising:
   administering to the individual at least one selected from the group consisting of antigenic proteins, antigenic peptides derived from the antigenic proteins, and DNAs capable of expressing the antigenic proteins or the antigenic peptides, in a therapeutically effective amount; and
   thereafter administering at least one selected from the group consisting of interferons and DNAs capable of expressing the interferons, in a therapeutically effective amount;
[8] a method for inducing antigen-specific T cell in an individual in need thereof, comprising:
   administering to the individual at least one selected from the group consisting of interferons and DNAs capable of expressing the interferons, in a therapeutically effective amount; and
   thereafter administering at least one selected from the group consisting of antigenic proteins, antigenic peptides derived from the antigenic proteins, and DNAs capable of expressing the antigenic proteins or the antigenic peptides, in a therapeutically effective amount.
[9] a method for enhancing induction of antigen-specific T cell in an individual in need thereof, comprising administering at least one selected from the group consisting of interferons and DNAs capable of expressing the interferons to the individual, in a therapeutically effective amount, wherein the individual has been treated by at least one selected from the group consisting of antigenic proteins, antigenic peptides derived from the antigenic proteins, and DNAs capable of expressing the antigenic proteins or the antigenic peptides;
[10] use of (1) at least one selected from the group consisting of antigenic proteins, antigenic peptides derived from the antigenic proteins, and DNAs capable of expressing the antigenic proteins or the antigenic peptides; and (2) at least one selected from the group consisting of interferons and DNAs capable of expressing the interferons for the manufacture of an agent for induction of antigen-specific T cell; and
[11] use of at least one of interferons and DNAs capable of expressing the interferons for the manufacture of an enhancing agent for induction of antigen-specific T cell.

### BRIEF DESCRIPTION OF THE DRAWINGS

Figure 1 is graphs each showing the action of enhancing CTL induction of IFN-α in an antigenic peptide administration system by using an osmotic pump, wherein Figure 1(A) shows the cytotoxic activity for peptide-pulsed EL-4 cells, and wherein Figure 1(B) shows the cytotoxic activity for peptide-nonpulsed EL-4 cells.
Figure 2 is graphs each showing the action of enhancing CTL induction of IFN-α in an IFA dosage form, wherein Figure 2(A) shows the cytotoxic activity for peptide-pulsed EL-4 cells, and wherein Figure 2(B) shows the cytotoxic activity for peptide-nonpulsed EL-4 cells.

The present invention provides an agent for induction of antigen-specific T cell comprising as active ingredients:
(1) at least one selected from the group consisting of antigenic proteins, antigenic peptides derived from the antigenic proteins, and DNAs capable of expressing the antigenic proteins or the antigenic peptides; and
(2) at least one selected from the group consisting of interferons and DNAs capable of expressing the interferons.

The present invention has been found on the basis of newly found action for enhancing induction of antigen-specific T cell owned by the interferon. When used in combination with the interferon, the induction of antigen-specific T cell can be more markedly enhanced, as compared with the case of the antigenic protein, the antigenic peptide derived from the antigenic protein, or a DNA capable of expressing the antigenic protein or the antigenic peptide alone. Such an effect exhibited by the use of the agent for induction of antigen-specific T cell of the present invention can neither be anticipated nor expected from the findings of the prior art.

The phrase "antigenic proteins, antigenic peptides derived from the antigenic proteins, and DNAs capable of expressing the antigenic proteins or the antigenic peptides" as used herein refers to any ones without particular limitation, as long as they are capable of inducing T cell that is specific for an antigenic peptide, and intends to encompass both of the following embodiments: ones capable of inducing antigen-specific T cell by directly forming a complex with MHC molecule (HLA molecule) on the cell surface; and ones capable of indirectly inducing antigen-specific T cell through the step comprising incorporating into cell an antigenic protein, an antigenic peptide, or a DNA capable of expressing the antigenic protein or the antigenic peptide, and thereafter binding to MHC molecule the expression product resulting from expression of the DNA or the antigenic peptide itself, or the intracellular degradation product of the expression product, the antigenic protein itself, or the like to form a complex, thereby presenting the complex to the cell surface.

The antigenic proteins include antigenic proteins derived from viruses, antigenic proteins derived from bacteria, tumor antigenic proteins, and the like. The antigenic proteins derived from viruses include antigenic proteins derived from viruses such as HIV, hepatitis C virus, hepatitis B virus, influenza virus, HPV, HTLV and EBV. The antigenic proteins derived from bacteria include antigenic proteins derived from bacteria such as *Mycobacterium tuberculosis.* A representative example of the tumor antigenic protein includes the ones listed in Table 1 in *Immunity* 10:281, 1999. Concretely, melanoma antigenic proteins, for example, include MAGE (*Science* 254:1643, 1991), gp 100 (*J. Exp. Med.* 179:1005, 1994), MART-1 (*Proc. Natl. Acad. Sci. U.S.A.* 91:3515, 1994) and tyrosinase (*J. Exp. Med.* 178:489, 1993); tumor antigenic proteins other than melanoma include tumor markers such as HER2/neu (*J. Exp. Med.* 181:2109, 1995), CEA (*J. Natl. Cancer Inst*. 87:982, 1995) and PSA *(J. Natl. Cancer Inst.* 89:293, 1997); and SART-1 derived from squamous cell carcinoma (*J. Exp. Med.* 187, 277-288, 1998; WO 97/46676); cyclophilin B *(Proc. Natl. Acad. Sci. U.S.A.* 88:1903, 1991), and the like. These antigenic proteins may be full-length polypeptides, partial polypeptides or variants thereof, as long as they are capable of inducing antigenic peptide-specific T cell.

The antigenic peptide derived from the antigenic protein (hereinafter simply referred to as "antigenic peptide") encompasses peptides comprising a part of the antigenic protein mentioned above, each of which comprises about 8 to about 20 amino acid residues, or derivatives which are functionally equivalent thereto, or polytopes resulting from linking two or more of the peptides or the derivatives thereof. The phrase "derivatives which are functionally equivalent thereto" as used herein refers to variants resulting from substitution, deletion and/or addition (including addition of amino acids to N-terminal or C-terminal of the peptide) of one or more amino acids in the amino acid sequence of the antigenic peptide, the variants being capable of inducing antigenic peptide-specific T cell.

Concretely, the tumor antigenic peptides include the following: The tumor antigenic peptides derived from SART-1 include HLA-A26-restricted tumor antigenic peptides comprising the amino acid sequence as shown in any one of SEQ ID NO: 1 to SEQ ID NO: 3; HLA-A24-restricted tumor antigenic peptides comprising the amino acid sequence as shown in any one of SEQ ID NO: 4 to SEQ ID NO: 9; and HLA-A2-restricted tumor antigenic peptides comprising the amino acid sequence as shown in any one of SEQ ID NO: 10 to SEQ ID NO: 15. The tumor antigenic peptides derived from cyclophilin B include HLA-A24-restricted tumor antigenic peptides comprising the amino acid sequence as shown in SEQ ID NO: 16 or SEQ ID NO: 17. The tumor antigenic peptides derived from SART-3 include HLA-A24-restricted tumor antigenic peptides comprising the amino acid sequence as shown in any one of SEQ ID NO: 18 to SEQ ID NO: 24. The tumor antigenic peptides derived from ART-1 include HLA-A24-restricted tumor antigenic peptides comprising the amino acid sequence as shown in any one of SEQ ID NO: 25 to SEQ ID NO: 27. The peptides derived from yeast having activity for tumor antigenic peptide include HLA-A24-restricted peptides comprising the amino acid sequence as shown in SEQ ID NO: 28.

Examples of the antigenic peptides derived from viruses include a peptide comprising the amino acid sequence as shown in SEQ ID NO: 34, and the like.

Furthermore, derivatives that are functionally equivalent to the above-mentioned tumor antigenic peptides include peptide derivatives resulting from substitution of amino acids on the basis of the sequence regularity (motif) of antigenic peptide presented by binding to HLA antigen, if the sequence regularity has been known. Concretely, HLA-A24 binding motifs have been known to be peptides comprising 8 to 11 amino acids, of which amino acid of the 2nd position is tyrosine, phenylalanine, methionine or tryptophan, and C-terminal is phenylalanine, leucine, isoleucine, tryptophan or methionine (*J. Immunol*. 152, 3913, 1994; *Immunogenetics* 41:178, 1995; *J. Immunol*. 155:4307, 1994). In addition, as HLA-A2 binding motifs, the motifs shown in Table 1 below have been known *(Immunogenetics* 41, 178, 1995; *J. Immunol*. 155:4749, 1995).

**Table 1**

| Type of HLA-A2 | 2nd Amino Acid from N-terminal | C-Terminal Amino Acid |
|---|---|---|
| HLA-A0201 | L, M | V, L |
| HLA-A0204 | L | L |
| HLA-A0205 | V, L, I, M | L |
| HLA-A0206 | V, Q | V, L |
| HLA-A0207 | L | L |

| | | |
|---|---|---|
| (Lenght of peptide being 8 to 11 amino acids.) | | |

Therefore, examples of the tumor antigenic peptide derivatives include peptide derivatives resulting from subjecting the above antigenic peptides to amino acid substitutions acceptable for the motifs of the peptides. Concrete examples of the derivatives include tumor antigenic peptide derivatives derived from SART-1, having the amino acid sequence as shown in any one of SEQ ID NO: 29 to SEQ ID NO: 31, and tumor antigenic peptide derivatives derived from cyclophilin B, having the amino acid sequence as shown in SEQ ID NO: 32 or SEQ ID NO: 33.

The term "polytope" refers to a recombinant peptide resulting from linking a plurality of antigenic peptides (see, for example, *the Journal of Immunology* 160, 1717, 1998). In the present invention, the polytope refers to a peptide comprising an amino acid sequence in which one or more kinds of the above-mentioned antigenic peptides or derivatives thereof are appropriately combined. The polytope can be obtained by inserting a recombinant DNA into an appropriate expression vector, the recombinant DNA being prepared by linking one or more kinds of genes encoding the above-mentioned antigenic peptides or derivatives thereof, and expressing the resulting recombinant vector in host cells.

The DNAs capable of expressing the antigenic proteins or the antigenic peptides include a DNA resulting from linking a gene encoding the above-mentioned antigenic protein or antigenic peptide to an appropriate expression vector. The gene may be genomic DNA, cDNA or chemically synthesized DNA. In addition, the gene may comprise a nucleotide sequence resulting from substitution, deletion, insertion or addition of one or more bases, as long as it encodes the antigenic protein or antigenic peptide.

The expression vectors may be any ones without particular limitation, as long as they can be expressed within antigen-presenting cells. The expression vectors include plasmid vectors and viral vectors. Preferably used plasmid vectors include commonly known vectors such as pCR3, pcDNA3.1, pRc/CMV2 (Invitrogen), pSPORT1, pSPORT2 and pSFV1 (GIBCO BRL). The viral vectors include, for example, vectors derived from DNA viruses or RNA viruses such as retrovirus, adenovirus, adeno-associated virus, herpesvirus, vaccinia virus, pox virus, poliovirus and Sindbis virus. Among these vectors, the retrovirus vector, adenovirus vector, adeno-associated virus vector and vaccinia virus vector are preferred.

The antigenic protein can be prepared by purifying by a conventional method a recombinant protein obtained by carrying out a cloning process for cDNA encoding the desired antigenic protein, a process for linking the cDNA to an expression vector, a process for introducing the vector to host cells, and a process for expressing the antigenic protein, in accordance with the basic textbooks such as *Molecular Cloning, Second Edition,* Cold Spring Harbor Laboratory Press (1989).

The antigenic peptide can be synthesized in accordance with the method used in ordinary peptide chemistry. Methods of synthesis include those described in the literatures *(Peptide Synthesis,* Interscience, New York, 1966; *The Proteins,* Vol. 2, Academic Press Inc., New York, 1976; *Pepuchido Gosei (Peptide Synthesis),* Maruzen, 1975; *Pepuchido Gosei No Kiso To Jikken (Basics and Experimentation of Peptide Synthesis),* Maruzen, 1985; *Iyakuhin No Kaihatsu, Zoku (Development of Pharmaceuticals, Sequel)*, Vol. 14, *Pepuchido Gosei (Peptide Synthesis),* Hirokawa Shoten, 1991). In addition, the antigenic peptide may also be prepared by purifying by a conventional method a recombinant peptide resulting from expression of a gene encoding the antigenic peptide, in accordance with *Molecular Cloning* mentioned above.

The DNA capable of expressing the antigenic protein or the antigenic peptide can be prepared by obtaining a recombinant DNA encoding the antigenic protein or antigenic peptide, and inserting the resulting recombinant DNA into an expression vector, in accordance with the basic textbooks such as *Molecular Cloning* mentioned above.

The interferons and the DNAs capable of expressing the interferons contained as another active ingredient in the agent for induction of antigen-specific T cell of the present invention may be any ones without particular limitation, as long as they enhance the induction of antigenic peptide-specific T cell. Examples thereof include interferon-α (IFN-α), interferon-β (IFN-β), interferon-γ (IFN-γ), and DNAs capable of expressing each interferon. Among them, IFN-α is preferable, from the viewpoint of having a great enhancing action for induction of antigen-specific T cell.

IFN-α may be natural-occurring ones, or ones obtained by gene recombination. The natural-occurring IFN-α products include commercial products available under the trade names "Sumiferon" (Sumitomo Pharmaceuticals), "IFNα Mochida" (Mochida Pharmaceutical) and "OIF" (Otsuka Pharmaceutical). IFN-α products obtained by genetic recombination include commercial products available under the trade names "Canferon" (Takeda Chemical Industries), "Roferon" (Roche) and "Intron" (Schering-Plough). Also included are consensus interferon (Amgen) and PEG-interferon (WO 99/64016). In addition, when laboratory animals are used, in the case of a mouse, for example, there may be used commercial products such as IFN-α, murine, Recombinant, *E. coli* (Calbiochem), IFN-α, Mouse, Recombinant, CHO cells (Hycult Biotechnology), IFN-α, Mouse, Rec. (Pestka Biomedical) and interferon-α, Mouse (Paesel).

The DNAs capable of expressing the interferons include a DNA resulting from linking a gene encoding the interferon to an appropriate expression vector. The gene may be genomic DNA, cDNA or chemically synthesized DNA. In addition, the gene may comprise a nucleotide sequence resulting from substitution, deletion, insertion or addition of one or more bases in the nucleotide sequence, as long as the gene encodes the interferon. Incidentally, the nucleotide sequences of the interferons are known in the art [See *Nature* 295, 503-508 (1982); *Nature* 284, 316-320 (1980);. *Nature* 290, 20-26 (1981); and *Nucleic Acids Res.* 8, 4057-4074 (1980); sequences registered at GenBank database, and the like].

The expression vectors may be any ones without particular limitation, as long as they can be expressed within a living body, and include plasmid vectors and viral vectors. Concrete examples thereof include those plasmid vectors and viral vectors exemplified above.

The DNA capable of expressing the interferon can be prepared by obtaining a recombinant DNA encoding the interferon, and inserting the resulting recombinant DNA into an expression vector, in accordance with the basic textbooks such as *Molecular Cloning* mentioned above.

Among the active ingredients contained in the agent for induction of antigen-specific T cell of the present invention, at least one is selected from the group consisting of the above-mentioned antigenic proteins, antigenic peptides and DNAs capable of expressing the antigenic proteins or the antigenic peptides. According to the purpose of treatment, two or more antigenic proteins, two or more antigenic peptides, or two or more of the DNAs may be selected.

Combinations of active ingredients contained in the agent for induction of antigen-specific T cell of the present invention include an antigenic protein with IFN-α, an antigenic protein with IFN-β, an antigenic protein with IFN-γ, an antigenic peptide with IFN-α, an antigenic peptide with IFN-β, an antigenic peptide with IFN-γ, a DNA capable of expressing the antigenic protein or the antigenic peptide with IFN-α, a DNA capable of expressing the antigenic protein or the antigenic peptide with IFN-β, a DNA capable of expressing the antigenic protein or the antigenic peptide with IFN-γ, and the like, and those combinations exemplified above in which each interferon is substituted by a DNA capable of expressing the interferon. The combinations of an antigenic protein with IFN-α and an antigenic peptide with IFN-α are preferable, from the viewpoint that enhancement of induction of antigen-specific T cell can be carried out more specifically and effectively.

The amount of the antigenic protein or antigenic peptide in the agent for induction of antigen-specific T cell of the present invention is not subject to particular limitation, as long as antigen-specific T cell is inducible. It is preferable that the amount of the antigenic protein or antigenic peptide is usually 0.0001 to 1000 mg, preferably 0.001 to 100 mg, and more preferably 0.01 to 10 mg per administration.

The amount of the DNA capable of expressing the antigenic protein or antigenic peptide in the agent for induction of antigen-specific T cell of the present invention is not subject to particular limitation, as long as antigen-specific T cell is inducible. The amount of the DNA is preferably 0.0001 to 100 mg, more preferably 0.001 to 10 mg per administration.

The amount of the interferon in the agent for induction of antigen-specific T cell of the present invention is not subject to particular limitation, as long as antigen-specific T cell is inducible. It is preferable that the amount of the interferon is about 10 U to about 1 x 10⁸ U per administration, for any of IFN-α, IFN-β and IFN-γ. In addition, the amount of the DNA capable of expressing the interferon is not subject to particular limitation, as long as the same level of effects is exhibited as those exhibited by the interferon in the amount exemplified above.

The ratio of the amounts of the antigenic protein, the antigenic peptide derived from the antigenic protein, the DNA capable of expressing the antigenic protein or the antigenic peptide, the interferon, and the DNA capable of expressing the interferon in the agent for induction of antigen-specific T cell of the present invention is not subject to particular limitation. Each active ingredient may be contained in the agent such that each active ingredient can be administered in the amount mentioned above, per administration of the agent.

The agent for induction of antigen-specific T cell of the present invention may further comprise an immunopotentiator. The term "immunopotentiator" as used herein refers to a substance possessing a so-called adjuvant activity which enhances cellular immunity induction specific to an antigen, when administered together with the antigen. In the present invention, the immunopotentiator may also include cytokines other than the interferons. The immunopotentiators include, for example, the adjuvants described in the publication (*Clin. Microbiol. Rev*. 7:277, 1994), concretely aluminum compounds, *C. parvum* derived from bacteria, BCG-CWS, lipopolysaccharide (LPS) or muramyl dipeptide (MDP), saponin derived from plants, and the like. The cytokines other than the interferon include GM-CSF, IL-12, IL-2, and the like.

It is preferable that the agent for induction of antigen-specific T cell of the present invention is in a preparation form so as to exhibit the desired pharmacological effect. Preparation forms suitable for this purpose include water-in-oil (w/o) emulsion preparations, oil-in-water (o/w) emulsion preparations, water-in-oil-in-water (w/o/w) emulsion preparations, liposome preparations, microsphere preparations, microcapsule preparations, solid injection preparations, and liquid preparations.

The water-in-oil (w/o) emulsion preparation takes the form in which active ingredients are dispersed in a water dispersion phase.

The oil-in-water (o/w) emulsion preparation takes the form in which active ingredients are dispersed in a water dispersion medium.

The water-in-oil-in-water (w/o/w) emulsion preparation takes the form in which active ingredients are dispersed in the innermost water dispersion phase.

The above emulsions can be prepared by referring to, for example, Japanese Patent Laid-Open Nos. Hei 8-985, Hei 9-122476, and the like.

The liposome preparation comprises fine particles taking the form in which active ingredients are enveloped in an aqueous phase or a membrane by means of a liposome having a lipid bilayer structure. Principal lipids for making liposome include phosphatidylcholine, sphingomyelin, and the like, to which lipids dicetyl phosphate, phosphatidic acid, phosphatidylserine, or the like, is added to charge and stabilize the liposome. Methods of making the liposome include ultrasonication method, ethanol injection method, ether injection method, reverse-phase evaporation method and French press extraction method.

The microsphere preparation comprises a homogenous polymeric matrix, and it is composed of fine particles taking the form in which active ingredients are dispersed in the matrix. Materials for the matrix include biodegradable polymers such as albumin, gelatin, chitin, chitosan, starch, polylactic acid and polyalkylcyanoacrylates. Methods of making the microsphere preparation may be those in accordance with the commonly known methods without particular limitation.

The microcapsule preparation is composed of fine particles taking the form in which active ingredients as the core substances are coated with a coating substance. The materials used for the coating substance include membrane-forming polymers such as carboxymethyl cellulose, cellulose acetate phthalate, ethyl cellulose, gelatin, gelatin-gum arabic, nitrocellulose, polyvinyl alcohol and hydroxypropyl cellulose. Methods of making the microcapsule preparation include coacervation method and interfacial polymerization method.

The solid injection preparation takes a preparation form in which active ingredients are sealed in a substrate made of collagen, silicon or the like, and solidified. Methods of making the solid injection preparation include a method described in publications such as *Pharm. Tech. Japan 7,* 402-409 (1991).

The liquid preparation takes the form in which active ingredients are mixed with pharmaceutically acceptable solvents, carriers, and the like. The pharmaceutical acceptable solvents include water, glucose solutions, physiological saline, and the like. Furthermore, pharmaceutical acceptable auxiliaries, for example, pH regulators or buffers, tension regulators, wetting agents, and the like may be contained.

The agent for induction of antigen-specific T cell of the present invention described above may be previously formed into a preparation or freshly prepared when administered to a patient. In other words, an antigenic protein, an antigenic peptide, a DNA capable of expressing the antigenic protein or the antigenic peptide, an interferon, a DNA capable of expressing the interferon, an immunopotentiator, and the like, which are active ingredients of the agent for induction of antigen-specific T cell of the present invention, and the preparation form emulsion and the like, may be previously mixed and formed into a preparation, or freshly prepared when administered to a patient.

The induction ability of antigen-specific T cell can be evaluated as described below for the agent for induction of antigen-specific T cell of the present invention prepared as described above.

The agent for induction of antigen-specific T cell of the present invention is one or more times administered to a laboratory animal. After 1 week from final administration, the spleen is extirpated, to prepare splenic lymphocytes. Splenocytes from an unsensitized mouse are also prepared, and pulsed for several hours with an antigenic peptide. Thereafter, the pulsed splenocytes are irradiated with an X-ray at about 2000 to about 5000 rad, and these cells are used as the antigen-presenting cells. By adding the antigen-presenting cells to lymphocytes from the immunized mouse, re-stimulation with the antigenic peptide is carried out in a culture system. The same stimulation is carried out plural times at a frequency of once a week as needed. After 1 week from final stimulation, lymphocytes are collected. The induction ability of antigen-specific T cell can be evaluated, for instance, by quantifying various cytokines (for example, IFN-γ) produced in response to antigenic peptide-specific T cell induced in the lymphocytes, with antigenic peptide-pulsed cells, antigen-positive cells, and the like as target cells, or by determining the cytotoxic activity of antigenic peptide-specific T cell on ⁵¹Cr-labeled target cells by the ⁵¹Cr release determination method (*J. Immunol*. **139**:2888, 1987). In the case of human, the induction ability of antigenic peptide-specific T cell can be evaluated in a similar method using peripheral blood monocytes (PBMC) separated from peripheral blood by the Ficoll method or the like in place of the splenic lymphocytes from a laboratory animal.

When the agent for induction of antigen-specific T cell of the present invention is administered to an antigen-positive patient, the antigenic peptide is presented at high density to the HLA antigen of antigen-presenting cells, so that the presented HLA antigen complex-specific T cell grows to destroy target cells (antigen-peptide-positive cells) or to produce various cytokines, whereby immunity can be activated. Preferably, the agent for induction of antigen-specific T cell of the present invention is used for treatment or prophylaxis of a tumor or viral infectious disease. When used for treatment or prophylaxis of a tumor, the agent for induction of antigen-specific T cell of the present invention comprising a tumor-specific tumor antigenic peptide as an active ingredient is administered to a patient, so that the cellular immunity specific to cancer cells is enhanced, whereby the tumor can be treated, or growth and metastasis of the tumor can be prevented. Furthermore, the agent for induction of antigen-specific T cell of the present invention may be used in combination with conventional chemotherapy or radiotherapy, whereby achieving a greater therapeutic effect.

When used for treatment or prophylaxis of a viral infectious disease, the agent for induction of antigen-specific T cell of the present invention comprising an antigenic peptide derived from virus as an active ingredient is administered to a patient, so that cellular immunity specific to virus-infected cells is enhanced, whereby the viral infectious disease can be treated or prevented.

Methods of administration include subcutaneous injection, persistent subcutaneous injection, intracutaneous injection, intravenous injection, intraarterial injection, intramuscular injection, topical injection and intraperitoneal administration. Continuous gradual administration using an osmotic pump or the like and implantation of a sustained-release preparation (for example, mini-pellet preparation) can be carried out. The frequency of administration is not subject to particular limitation, and it is preferably once per several days to several months.

Furthermore, the present invention also provides an enhancing agent for induction of antigen-specific T cell comprising at least one of interferons and DNAs capable of expressing the interferons as an active ingredient. As described above, the interferon, which is an active ingredient of the agent for induction of antigen-specific T cell of the present invention, exhibits an effect for enhancing the induction of antigen-specific T cell. Therefore, in addition to its use as a component of the agent for induction, the interferon can also be used alone as an enhancing agent for induction for increasing the induction activity for antigen-specific T cell. The enhancing agent for induction of the present invention is effectively used, for example, in cases where T cell induction activity with vaccine is insufficient. It is especially preferable to use interferon-α.

The amount of the interferon in the enhancing agent for induction of the present invention is not subject to particular limitation, as long as antigen-specific T cell induction can be enhanced. The amount of the interferon is about 10 U to about 1 x 10⁸ U per administration, for any of IFN-α, IFN-β and IFN-γ. In addition, the amount of the DNA capable of expressing the interferon is not subject to particular limitation, as long as the same level of effects is exhibited as those exhibited by the interferon in the amount exemplified above.

The method of preparing the enhancing agent for induction of the present invention and the method of administering the enhancing agent for induction are the same as those for the above-mentioned agent for induction of antigen-specific T cell.

The method for induction of antigen-specific T cell in human in the present invention is not subject to particular limitation. Embodiments of such a method include:
(1) administration of at least one selected from the group consisting of antigenic proteins, antigenic peptides derived from the antigenic proteins, DNAs capable of expressing the antigenic proteins or the antigenic peptides; and
(2) administration of at least one selected from the group consisting of interferons and DNAs capable of expressing the interferons, by administering a preparation comprising both components, or by administering the both components as separate preparations. In the case of using separate preparations, the two components may be administered simultaneously or sequentially. When administered sequentially, either of the two components may be administered beforehand, and either may be administered afterwards. An example of such a method includes a method comprising administering at least one selected from the group consisting of antigenic proteins, antigenic peptides derived from the antigenic proteins, DNAs capable of expressing the antigenic proteins or the antigenic peptides; and thereafter administering at least one of interferons and DNAs capable of expressing the interferons. In addition, the interval for administration may be immediately after, or about one day to six months after, administering the first preparation. Alternatively, a further embodiment comprises administering at least one of interferons and DNAs capable of expressing the interferons to an individual who has been administered with at least one selected from the group consisting of antigenic proteins, antigenic peptides derived from the antigenic proteins, DNAs capable of expressing the antigenic proteins or the antigenic peptides. In this embodiment, there is encompassed an embodiment where an interferon or the like is administered regularly to a patient who has been administered with an antigenic protein or the like.

### EXAMPLES

The present invention is hereinafter described by means of the following examples, without intending to limit the scope or spirit of the present invention thereto.

### Example 1

### Action of Enhancing CTL Induction of IFN-α in Antigenic Peptide Administration System by Using Osmotic Pump

CTL induction was examined in a system in which an osmotic pump (Alza, Model "1003D") was charged with the drug and subcutaneously implanted in a mouse to release the drug over a 3-day period. The following five experimental groups were set:
Group 1: 100 µg of peptide Flu₃₆₆₋₃₇₄ was administered using the osmotic pump.
Group 2: 10⁵ units (hereinafter abbreviated U) of IFN-α was administered using the osmotic pump.
Group 3: 100 µg of peptide Flu_{366·374} and 10⁵ U of IFN-α were administered using the osmotic pump.
Group 4: 100 µg of peptide Flu₃₆₆₋₃₇₄ as mixed with incomplete Freund's adjuvant was administered.
Group 5: Not treated (without drug administration).

The amino acid sequence of peptide Flu₃₆₆₋₃₇₄, which is an H-2D^{b} restrictive antigen peptide derived from influenza virus, is Ala-Ser-Asn-Glu-Ser-Met-Glu-Thr-Met (SEQ ID NO: 34), and the peptide was synthesized by the Fmoc method.

Incomplete Freund's adjuvant (hereinafter abbreviated "IFA") was purchased from Wako Pure Chemical Industries. An emulsion was freshly prepared before use by connecting two glass syringes and mixing an equal volume of IFA and a peptide solution (2 mg/ml).

Mouse IFN-α was prepared by infecting the mouse cell line EAT cells (derived from Ehrlich ascites carcinoma, ATCC Stock Number CCL-77), previously treated with sodium butyrate, with the Newcastle disease viruses (hereinafter abbreviated "NDV"), treating the infected cells with theophylline to produce IFN-α, and purifying the resulting IFN-α using a CPG column and anti-IFN-α antibody column. IFN-α titer was determined by the measurement method with inhibition of viral cytopathic effect (CPE) by IFN-α as an index with reference to the method described in a book (M.J. Clemens et al., edts., *Lymphokines and Interferons: a practical approach, p.* 6, IRL Press, Oxford, 1987).

For Groups 1 to 3, the drug-filled osmotic pump was subcutaneously implanted at the tail base of C57BL/6 mouse (Charles River Japan). For Group 4, 0.1 ml of an IFA emulsion was subcutaneously administered to the tail base of C57BL/6 mouse. For each group, three mice were used.

After 7 days from the drug administration, the spleen was extirpated from the mice in each group and subjected to hemolytic treatment to prepare splenocytes. The splenocytes were sown to 10 wells of a 24-well plate at 5 x 10⁶ cells/well (1.7 ml). Splenocytes for antigen-presenting cells were prepared from non-treated mouse spleen, and peptide-pulsed by adding 90 µg/ml of the peptide Flu₃₆₆₋₃₇₄, and culturing the mixture at 37°C for 1 hour. Thereafter, the peptide-pulsed splenocytes were irradiated with an X-ray (2000 rad). These resulting antigen-presenting cells were added to the above 24-well plates sown with the mouse splenocytes for Groups 1 to 5 at 5 x 10⁶ cells/well (0.1 ml), to conduct re-stimulation with the peptide *in vitro.* In the culture, there was employed a culture medium of RPMI 1640 supplemented with 10% FCS, 10 U/ml mouse IL-2 (BECTON DICKINSON), 10 mM HEPES, 20 mM L-glutamine, 1 mM sodium pyruvate, 1 mM MEM non-essential amino acids (GIBCO BRL), 1% MEM vitamins (GIBCO BRL) and 55 µM 2-mercaptoethanol.

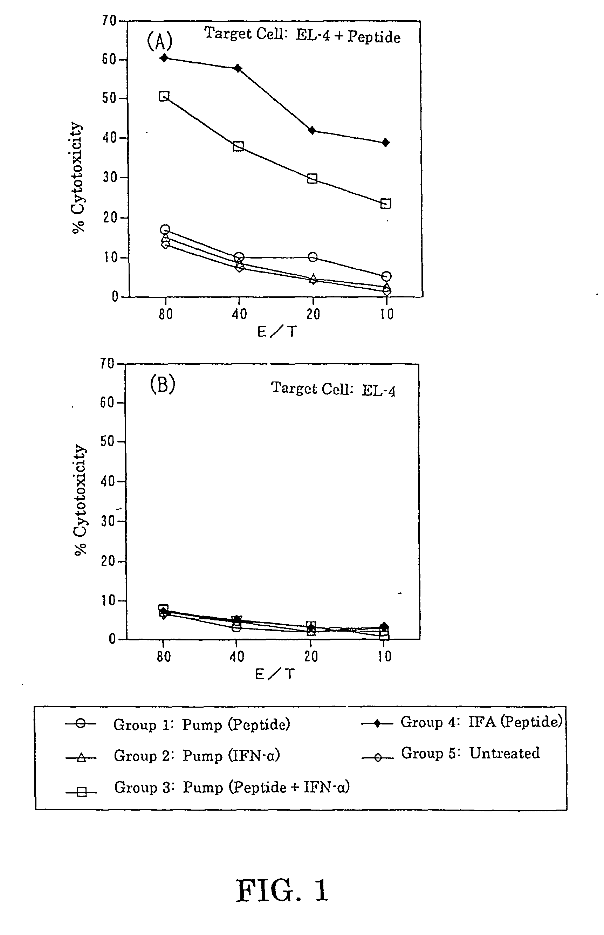

After 5 days of culture, the splenocytes were collected from the plates and subjected to the ⁵¹Cr release determination method in accordance with the method described in T. Nishimura et al., *J. Immunol.,* 139, 2888 (1987) to determine the peptide-specific cytotoxic activity. The target cells used for determination of the peptide-specific cytotoxic activity were EL-4 cells (derived from lymphoma, ATCC Stock Number TIB-39) labeled with ⁵¹Cr and pulsed with the peptide Flu_{366·374}. The target cells used for determination of the non-specific cytotoxic activity were EL-4 cells labeled with ⁵¹Cr. The results are shown in Fig. 1(A) and Fig. 1(B). As shown in Fig. 1(B), the cytotoxic activity for peptide-nonpulsed EL-4 cells was as low as 10% or less in all groups; in the present example, non-specific killer cells were not induced. Fig. 1(A) shows the cytotoxic activity for peptide-pulsed EL-4 cells. As shown in Fig. 1(A), no peptide-specific CTL was induced when the peptide or IFN-α was administered alone using the osmotic pump (Groups 1 and 2), as the case of "not treated" (Group 5). When IFN-α was administered simultaneously with the peptide by using the osmotic pump, however, the peptide-specific cytotoxic activity was detected as the case of IFA administration. Therefore, it was clarified that CTL was induced. It has been clarified from the results that IFN-α possesses the action of enhancing CTL induction.

### Example 2

### Action of Enhancing CTL Induction of IFN-α in IFA Preparation Form

The action of IFN-α in a system for inducing specific CTL by administering an antigenic peptide in an IFA emulsion preparation form was evaluated.
Group 1: 100 µg of peptide Flu₃₆₆₋₃₇₄ was administered in the IFA preparation form.
Group 2: 100 µg of peptide Flu_{366·374} and 10⁵ U of IFN-α were administered in the IFA preparation form.
Group 3: The IFA preparation form (vehicle) was administered.

The peptide Flu_{366·374}, IFA and IFN-α used were of the same lots as those used in Example 1. An emulsion was freshly prepared before use by connecting two glass syringes and mixing an equal volume of the drug solution prepared with PBS and IFA. This emulsion in an amount of 0.1 ml was subcutaneously administered to the tail base of C57BL/6 mouse. For each group, three mice were used. After 7 days from the drug administration, splenocytes were prepared, and thereafter re-stimulation was carried out with the peptide in the same manner as in Example 1. After 5 days of culture, the cytotoxic activity was determined by the ⁵¹Cr release method.

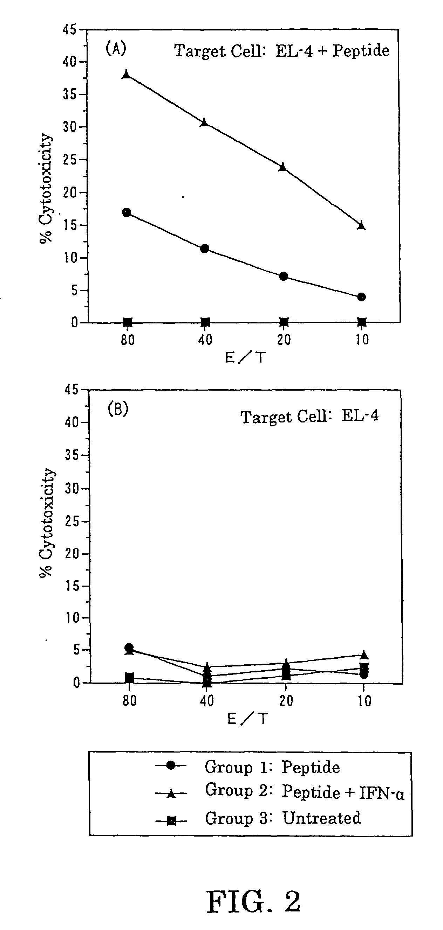

The results are shown in Fig. 2(A) and Fig. 2(B). As shown in Fig. 2(B), the cytotoxic activity for peptide-nonpulsed EL-4 cells was as low as 10% or less in all groups; in the present example, non-specific killer cells were not induced. Fig. 2(A) shows the cytotoxic activity for peptide-pulsed EL-4 cells. As shown in Fig. 2(A), more potent cytotoxic activity was induced in the group subjected to administration of the peptide and IFN-α (Group 2) than in the group subjected to administration of the peptide alone (Group 1), in the IFA form. It was clarified from the results that IFN-α exhibits the action of enhancing induction of peptide-specific CTL induction even in the IFA preparation form.

In the agent for induction of antigenic-specific T cell of the present invention, since interferons such as IFN-α, which are active ingredients, enhance the action of antigenic peptide-specific T cell induction, the agent is useful as a vaccine purposed for induction of antigenic peptide-specific cellular immunity. In one embodiment the invention provides use of antigenic proteins, antigenic peptides derived from the antigenic proteins or DNAs capable of expressing said antigenic proteins or said antigenic peptides in the manufacture of a medicament for induction of antigenic-specific T cells or enhancing the induction of antigen-specific T cells, characterised in that the medicament also comprises an interferon or a DNA capable of expressing said interferon.

### FREE TEXT FOR SEQUENCE LISTING

SEQ ID NO: 29 is a designed peptide based on the motif for antigenic peptide from SART-1, to which HLA antigen binds.
SEQ ID NO: 30 is a designed peptide based on the motif for antigenic peptide from SART-1, to which HLA antigen binds.
SEQ ID NO: 31 is a designed peptide based on the motif for antigenic peptide from SART-1, to which HLA antigen binds.
SEQ ID NO: 32 is a designed peptide based on the motif for antigenic peptide from cyclophilin B, to which HLA antigen binds.
SEQ ID NO: 33 is a designed peptide based on the motif for antigenic peptide from cyclophilin B, to which HLA antigen binds.

## Claims

1. Use of at least one selected from the group consisting of interferons and DNAs capable of expressing said interferons in the manufacture of an agent for enhancing the induction of antigen-specific T cells.

2. Use according to claim 1, wherein said interferon is interferon-α.

3. Use of at least one selected from the group consisting of interferons and DNAs capable of expressing said interferons in a therapeutically effective amount in the manufacture of a medicament for inducing antigen-specific T cells in an individual in need thereof, wherein said individual has been administered with at least one selected from the group consisting of antigenic proteins, antigenic peptides derived from the antigenic proteins, and DNAs capable of expressing said antigenic proteins or said antigenic peptides in a therapeutically effective amount, and optionally wherein said interferon is interferon-α.

4. Use according to claim 3 wherein the antigen is a tumor antigen or an antigen derived from virus.

5. Use of at least one selected from the group consisting of antigenic proteins, antigenic peptides derived from the antigenic proteins, and DNAs capable of expressing said antigenic proteins or said antigenic peptides in a therapeutically effective amount in the manufacture of a medicament for inducing antigen-specific T cells in an individual in need thereof, wherein said individual has been administered with at least one selected from the group consisting of interferons and DNAs capable of expressing said interferons, and optionally wherein said interferon is interferon-α.

6. Use according to claim 5 wherein the antigen is a tumor antigen or an antigen derived from virus.

7. A product containing at least one selected from the group consisting of antigenic proteins, antigenic peptides derived from the antigenic proteins, and DNAs capable of expressing said antigenic proteins or said antigenic peptides and at least one selected from the group consisting of interferons and DNAs capable of expressing said interferons for simultaneous, separate or sequential use in the induction of antigen-specific T cells, and optionally wherein said interferon is interferon-α.

8. A product according to claim 7 wherein the antigen is a tumor antigen or an antigen derived from virus.

9. Use of at least one selected from the group consisting of interferons and DNAs capable of expressing said interferons to the individual, in a therapeutically effective amount, in the manufacture of a medicament for enhancing the induction of antigen-specific T cells in an individual who has been treated by at least one selected from the group consisting of antigenic proteins, antigenic peptides derived from the antigenic proteins, and DNAs capable of expressing said antigenic proteins or said antigenic peptides.

10. Use of
(1) at least one selected from the group consisting of antigenic proteins, antigenic peptides derived from the antigenic proteins, and DNAs capable of expressing said antigenic proteins or said antigenic peptides; and
(2) at least one selected from the group consisting of interferons and DNAs capable of expressing said interferons, in the manufacture of an agent for induction of antigen-specific T cells.

11. Use according to claim 9, wherein said interferon is interferon-α.

12. Use according to claim 10, wherein (1) comprises an antigenic protein or antigenic peptide.

13. Use according to any one of claims 10 to 12 wherein the agent further comprises an immunopotentiator.

14. Use according to any one of claims 10 to 13, wherein the agent is in a preparation form selected from the group consisting of water-in-oil (w/o) emulsion preparations, oil-in-water (o/w) emulsion preparations, water-in-oil-in-water (w/o/w) emulsion preparations, liposome preparations, microsphere preparations, microcapsule preparations, solid injection preparations and liquid preparations.

15. Use according to any one of claims 10 to 14, wherein the antigen is a tumor antigen or an antigen derived from virus.

16. Use according to any one of claims 10 to 15 wherein the agent is for treatment or prophylaxis of a tumor or a viral infectious disease.
